# EUROPEAN PATENT SPECIFICATION

(11) **EP 3 263 697 B1**
(45) Date of publication and mention of the grant of the patent: **31.03.2021**
(21) Application number: 16755730.5
(22) Date of filing: 26.02.2016
(51) Int. Cl.: C12N 5/0775

(54) **CULTURE MEDIUM FOR CULTURING MESENCHYMAL STEM CELL, METHOD FOR CULTURING MESENCHYMAL STEM CELL, AND MESENCHYMAL STEM CELL**
KULTURMEDIUM ZUR KULTIVIERUNG MESENCHYMALER STAMMZELLEN, VERFAHREN ZUR KULTIVIERUNG MESENCHYMALER STAMMZELLEN UND MESENCHYMALE STAMMZELLE
MILIEU DE CULTURE POUR CULTIVER UNE CELLULE SOUCHE MÉSENCHYMATEUSE, PROCÉDÉ POUR CULTIVER UNE CELLULE SOUCHE MÉSENCHYMATEUSE ET CELLULE SOUCHE MÉSENCHYMATEUSE

(30) Priority: 27.02.2015 US 201562121513 P
(43) Date of publication of application: 03.01.2018
(73) Proprietor: Rohto Pharmaceutical Co., Ltd., Osaka-shi Osaka 544-8666 (JP)
(72) Inventor: IKEYAMA, Yoshifumi, Osaka-shi Osaka 544-8666 (JP); OKUBO, Toru, Osaka-shi Osaka 544-8666 (JP); NISHIDA, Hiroyuki, Osaka-shi Osaka 544-8666 (JP); TSUDA, Tomohiro, Osaka-shi Osaka 544-8666 (JP); UNO, Eiko, Osaka-shi Osaka 544-8666 (JP); YUMOTO, Masayo, Osaka-shi Osaka 544-8666 (JP)
(74) Representative: Mewburn Ellis LLP
(86) International application number: PCT/JP2016/055930
(87) International publication number: WO 2016/136986

(56) References cited:
- WO-A1-2007/080919
- WO-A1-2008/133904
- JP-A- 2006 325 444
- JP-A- 2008 061 569
- JP-A- 2010 504 090
- JP-A- 2010 524 499
- JP-A- 2012 520 672
- JP-A- 2014 516 562
- D STANCO ET AL: "Donor-matched mesenchymal stem cells from knee infrapatellar and subcutaneous adipose tissue of osteoarthritic donors display differential chondrogenic and osteogenic commitment", EUROPEAN CELLS AND MATERIALS, vol. 27, 23 April 2014 (2014-04-23), pages 298-311, XP55489060, DOI: 10.22203/eCM.v027a21
- XU BAIYAO ET AL: "Role of p38, ERK1/2, focal adhesion kinase, RhoA/ROCK and cytoskeleton in the adipogenesis of human mesenchymal stem cells", JOURNAL OF BIOSCIENCE AND BIOENGINEERING, vol. 117, no. 5, May 2014 (2014-05), pages 624-631, XP002782756,
- LING L ET AL: "Wnt signaling controls the fate of mesenchymal stem cells", GENE, ELSEVIER, AMSTERDAM, NL, vol. 433, no. 1-2, 15 March 2009 (2009-03-15), pages 1-7, XP025940354, ISSN: 0378-1119, DOI: 10.1016/J.GENE.2008.12.008 [retrieved on 2009-02-11]
- DEANS ROBERT J ET AL: "Mesenchymal stem cells: Biology and potential clinical uses", EXPERIMENTAL HEMATOLOGY, ELSEVIER INC, US, vol. 28, no. 8, 1 August 2000 (2000-08-01) , pages 875-884, XP002201188, ISSN: 0301-472X, DOI: 10.1016/S0301-472X(00)00482-3

## Description

### TECHNICAL FIELD

The present disclosure is related to a mesenchymal stem cell culture medium, a method for culturing mesenchymal stem cells, and mesenchymal stem cells.

### BACKGROUND ART

Lately, a progress has been seen in the development of medicaments utilizing cells or tissues of a living body and studies on regenerative medicine which are receiving attention. Above all, researches on ES cells and iPS cells have been accelerated as organ regeneration techniques. On the other hand, cellular therapy which utilizes somatic stem cells such as bone marrow stem cells by extraction, utilizes the original function of the somatic stem cells that improves/repairs tissue disorders caused by diseases, is attracting particular attention among the topics in the field of regenerative medicine as having a better feasibility, and thus, studies on this have been promoted. Somatic stem cells, in general, cannot differentiate into all organs and tissues, but they differentiate into specific tissues and organs. There are various kinds of somatic stem cells, and mesenchymal stem cells are one of them.

The umbilical cord contains abundant somatic cells that can be the origin of various kinds of cells. For example, hematopoietic stem cells deriving from umbilical cord blood are also included therein, and they have already been used for the treatment of refractory blood diseases such as leukemia and aplastic anemia. Moreover, in addition to the above, the umbilical cord contains various stem cells that are cells classified into the category of mesenchymal stem cells and pluripotent stem cell-like cells that are capable of differentiating into various cells such as nerves, muscles, heart, blood vessels, bones, and skins.

While various media for culturing the research-use mesenchymal stem cells have been developed (for example, refer to Patent Document 1 and Patent Document 2); however, the development of a medium that allows the culturing of a more suitable stem cells for the treatment of diseases involving transplantation and the like so that the stem cells are cultured in a large amount, efficiently, and over a long period of time while maintaining the undifferentiating property has been awaited.

WO 2008/133904 describes methods for expanding stem cells comprising modulating a PTEN pathway and a Wnt pathway.

### PRIOR ART DOCUMENTS

### Patent Documents

Patent Document 1: US 20100015710 A1
Patent Document 2: JP 2010-094062 A

### DISCLOSURE OF THE INVENTION

### Problems to be solved by the Invention

The present invention has been carried out in view of the above-mentioned conventional techniques, and thus, aims to provide a medium that can culture mesenchymal stem cells in a large amount, efficiently, and over a long period of time while maintaining the undifferentiating property of the mesenchymal stem cell, and in particular, a mesenchymal stem cell culture medium more suitable for the culturing of umbilical cord mesenchymal stem cells among the mesenchymal stem cells.
The invention is as defined in the appended claims.

### Means for Solving the Problems

The present inventors have carried out dedicated studies to solve the above-mentioned problem, which resulted in the finding that the medium supplemented with a specific component added to a basal medium for culturing animal cells is appropriate for the culturing of the mesenchymal stem cells, The present disclosure can be summarized as the following.
(1) A mesenchymal stem cell culture medium comprising at least 2 kinds of components selected from the group consisting of a PTEN inhibitor, a p53 inhibitor, a p38 inhibitor, a Wnt signal activator and a ROCK inhibitor as well as basal medium for culturing animal cells;
(2) The mesenchymal stem cell culture medium according to (1) comprising at least 3 kinds of components selected from the group consisting of a PTEN inhibitor, a p53 inhibitor, a p38 inhibitor, a Wnt signal activator and a ROCK inhibitor, as well as basal medium for culturing animal cells;
(3) The mesenchymal stem cell culture medium according to (1) or (2) comprising the PTEN inhibitor, the p53 inhibitor, the p38 inhibitor, the Wnt signal activator and the ROCK inhibitor;
(4) The mesenchymal stem cell culture medium according to any one of (1) to (3) further comprising at least one kind of component selected from the group consisting of growth factors and steroidal compounds;
(5) The mesenchymal stem cell culture medium according to any one of (1) to (4), wherein the PTEN inhibitor is selected from the group consisting of VO-OHPic, HOPic and pV;
(6) The mesenchymal stem cell culture medium according to any one of (1) to (5), wherein the p53 inhibitor is selected from the group consisting of sodium orthovanadate, pifithrin-α and MDM2 protein;
(7) The mesenchymal stem cell culture medium according to any one of (1) to (6), wherein the p38 inhibitor is selected from the group consisting of SB203580, SB202190, BIRB796, LY2228820, VX-702, PH-797804, TAK-715, VX-745 and Skepinone-L;
(8) The mesenchymal stem cell culture medium according to any one of (1) to (7), wherein the Wnt signal activator is a LiCl or a complement molecule C1q;
(9) The mesenchymal stem cell culture medium according to any one of (1) to (8), wherein the ROCK inhibitor is selected from the group consisting of Y-27632, K-115 and Fasudil hydrochloride;
(10) A method for culturing mesenchymal stem cells comprising culturing mesenchymal stem cells using the mesenchymal stem cell culture medium according to any one of (1) to (9), and
(11) A mesenchymal stem cell which is CD29, CD73, CD90, CD105, and CD166 positive and is cultured in the mesenchymal stem cell culture medium according to any one of (1) to (9).

### Effects of the Invention

When mesenchymal stem cells are cultured using a mesenchymal stem cell culture medium of the present invention, the undifferentiating property would be maintained over a long period of time. Moreover, as compared to the conventional medium, the medium of the present invention allows a further efficient proliferation of the mesenchymal stem cells over a long period of time while maintaining a favorable cell condition and properties.

### BRIEF DESCRIPTION OF THE DRAWINGS

FIG. 1 is a photograph of umbilical cord mesenchymal stem cells and adipose derived stem cells at day 3 of culturing in the medium of the present disclosure.
FIG. 2 is a graph showing the proliferation of umbilical cord mesenchymal stem cells and adipose derived stem cells in the medium of the present disclosure.
FIG. 3 is a photograph of umbilical cord mesenchymal stem cells and adipose derived stem cells at day 8 of culturing in the medium of the present disclosure.
FIG. 4 is a figure showing the expression of a cell surface marker of the adipose derived stem cells at day 8 of culturing in the medium of the present disclosure.
FIG. 5 is a figure showing the expression of a cell surface marker of the umbilical cord mesenchymal stem cells at day 8 of culturing in the medium of the present disclosure.
FIG. 6 is a figure showing the expression of a cell surface marker of the umbilical cord mesenchymal stem cells at day 11 of culturing in the medium of the present disclosure.
FIG. 7 is a graph showing the proliferation of umbilical cord mesenchymal stem cells in the medium of the present disclosure.
FIG. 8 is a photograph of adipose derived stem cells at day 5 of culturing in the medium of the present disclosure.
FIG. 9 is a photograph of umbilical cord mesenchymal stem cells at day 5 of culturing in the medium of the present disclosure.
FIG. 10 is a figure showing the expression of a cell surface marker (CD105) of the umbilical cord mesenchymal stem cells at week 3 of culturing in the medium of the present disclosure.
FIG. 11 is a graph comparing the oxidative stress resistance of the obtained umbilical cord derived mesenchymal stem cells when cultured in the medium of the present disclosure and in the conventional medium.
FIG. 12 is a graph showing the proliferation of the umbilical cord mesenchymal stem cells in the medium of the present disclosure cultured for a long period of time.
FIG. 13 is a graph showing the proliferation of the umbilical cord mesenchymal stem cells in the medium of the present disclosure cultured for a long period of time.
FIG. 14 is a photograph showing the umbilical cord mesenchymal stem cells in the medium of the present disclosure cultured for a long period of time.
FIG. 15 is a graph showing the proliferation of the umbilical cord mesenchymal stem cells in the medium of the present disclosure.
FIG. 16 is a graph showing the proliferation of the umbilical cord mesenchymal stem cells in the medium of the present disclosure.

### MODES FOR CARRYING OUT THE METHODS

### Mesenchymal stem cell culture medium

The mesenchymal stem cell culture medium of the present disclosure contains at least 2 kinds of components selected from the group consisting of a PTEN inhibitor, a p53 inhibitor, a p38 inhibitor, a Wnt signal activator, and a ROCK inhibitor, and a basal medium for culturing animal cells. By containing these components, the medium allows the culturing of the mesenchymal stem cells while maintaining the undifferentiating property over a long period of time. Here, the word "over a long period of time" refers to 8 days or more, preferably 15 days or more, and more preferably 30 days or more, and even more preferably 50 days or more, and particularly preferably 60 days or more. Moreover, the medium allows the efficient proliferation of the mesenchymal stem cells while maintaining a favorable cell condition over a long period of time. In addition, whether the cell condition is favorable or not can be determined based on the morphology of the cell, proliferation speed, and the like, based on the common technical knowledge of those skilled in the art. Furthermore, as an unpredictable effect, by culturing in the medium of the present disclosure the oxidative stress resistance function of the mesenchymal stem cells would be improved. Preferably, the medium shall further contain at least one kind of component selected from the group consisting of a growth factor and a steroidal compound. Moreover, the medium may contain other components to the extent that the effect described herein would not be impeded. Hereinbelow, the medium is disclosed.

In the present specification, mesenchymal stem cells refer to cells that have the ability to differentiate into cells that belong to a group of mesenchymal cells (osteocytes, chondrocytes, adipocytes, and the like) and cells that can proliferate while maintaining the ability.

In the present specification, umbilical cord mesenchymal stem cells refer to stem cells deriving from umbilical cord, umbilical cord related tissues, and umbilical cord blood. More specifically, umbilical cord mesenchymal stem cells are mesenchymal stem cells deriving from umbilical cord and structures around the umbilical cord. In particular, they are a structure around the umbilical cord such as umbilical cord, placenta, amnion, chorion, decidua, fetal membrane, Wharton's Jelly, and mesenchymal stem cells deriving from umbilical cord blood. Among these, preferably, it is Wharton's Jelly, mesenchymal stem cells deriving from amnion, and more preferably, mesenchymal stem cells deriving from Wharton's Jelly. Furthermore, in the present specification, adipose stem cells or adipose derived stem cells refer to stem cells deriving from adipose tissues. Specifically, they are mesenchymal stem cells deriving from adipose tissues.

### PTEN inhibitors disclosure.

PTEN inhibitors in the present disclosure refer to all substances that have the function to inhibit the action of the PTEN (Phosphatase and Tensin Homolog Deleted from Chromosome 10) gene or PTEN protein. The PTEN gene is located on chromosome 10q23.3 and it is identified as a tumor suppression factor. PTEN protein is widely expressed throughout the cells of the entire body and it is known as an enzyme catalyzing the dephosphorylation reaction of phosphatidylinositol 3,4,5-trisphosphate; PIP3 which is an inositol phospholipid. Moreover, PIP3 is synthesized intracellularly by PI3 kinase (PI3K) and induces the activation of protein kinase B (PKB)/AKT. PTEN is responsible for the dephosphorylation reaction of this PIP3 and it is believed to have an action of converting PIP3 into a phosphatidylinositol 4,5-bisphosphate; PIP2. Therefore, PTEN negatively regulates the PI3K/AKT signal transduction pathway. When the activity of PTEN is inhibited, PIP3 accumulates in the cells, and thus, results in the activation of the PI3K/AKT signal transduction pathway.

The PTEN inhibitor of the present disclosure is preferably a compound containing vanadium, for example, the compound includes such as pV(phenbig)bis peroxo(phenyl biguanide)oxovanadium dipotassium) or HOpic(bpV)bis peroxo(5-hydroxypyridine-2-carboxy)oxovanadium dipotassium, VO-OHPic trihydrate((OC-6-45)Aqua(3-hydroxy-2-piperidinecarboxylato-kapaN1, kapaO2) [3-(hydroxy-kapaO)-2-piperidinecarboxylato(2-)-kapaO2]oxo-vanadate(1-), hydrogen trihydrate), and the like. Of these, VO-OHPic, HOpic, and pV are more preferable. A sufficient effect can be obtained from any of VO-OHPic, HOpic, and pV; however, pV is more preferable. These may be used alone, or two or more kinds may be used in combination.

The concentration of the medium of the PTEN inhibitor in the mesenchymal stem cell culture medium of the present disclosure is preferably 10 nM - 10 µM, more preferably 50 nM - 1 µM, further preferably 100 nM - 750 nM, and particularly preferably 250 nM - 750 nM,

### P53 inhibitors

The p53 inhibitor of the present disclosure refers to all substances having a function of inhibiting the action of p53 gene or p53 protein. p53 gene is located on chromosome 17p13.1, which is also known as a tumor suppressor gene. p53 protein has various physiological activities and acts as a transcription factor.

The p53 inhibitor includes sodium orthovanadate, pifithrin-α, MDM2 protein, and the like, for example. Of these, sodium orthovanadate, pifithrin-α, MDM2 protein are preferred. A sufficient effect can be obtained from any of sodium orthovanadate, pifithrin-α, and MDM2 protein; however, pifithrin-α is more preferable. These may be used alone, or two or more kinds may be used in combination.

The concentration of the p53 inhibitor in the mesenchymal stem cell culture medium of the present disclosure is preferably 100 nM - 1 mM, more preferably 500 nM - 500 µM, and further preferably 1 µM - 100 µM,

### p38 inhibitors

The p38 inhibitor of the present disclosure refers to all substances having a function of inhibiting the action of p38 gene or p38 protein. p38 is one of the MAP kinase which is a serine/threonine kinase (Mitogen-Activated Protein Kinase). It has been revealed that MAP kinase is involved in signaling molecules transmitting external stimuli, cell proliferation, differentiation, gene expression, apoptosis, and the like.

The p38 inhibitor includes SB203580(Methyl[4-[4-(4-fluorophenyl)-5-(4-pyridinyl)-1H-imidazol-2-yl]phenyl] sulfoxide), SB202190(4-[4-(4-Fluorophenyl)-5-(4-pyridinyl)-1H-imidazol-2-yl]phenol), BIRB796(Doramapimod;l-[5-tert-Butyl-2-(4-methylphenyl)-2H-pyrazole-3-yl]-3-[4-(2-morpholinoethoxy)-1-naphthyl]urea), LY2228820(5-[2-(1,1-Dimethylethyl)-5-(4-fluorophenyl)-1H-imidazol-4-yl]-3-(2,2-dimethylpropyl)-3H-imidazo[4,5-b]pyridin-2-amine dimethanesulfonate), VX-702(2-(2,4-Difluorophenyl)-6-[(2,6-difluorophenyl)(aminocarbonyl)amino]pyridine-3-carboxamide), PH-797804(N,4-Dimethyl-3-[3-bromo-4-[(2,4-difluorobenzyl)oxy]-6-methyl-2-oxo-1,2-dihydropyridine-1-yl]benzamide), TAK-715(N-[4-[2-Ethyl-4-(3-methylphenyl)thiazole-5-yl]-2-pyridyl]benzamide), VX-745(5-(2,6-Dichlorophenyl)-2-[2,4-difluorophenyl)thio]-6H-pyrimido[1,6-b]pyridazin-6-one), Skepinone-L((2R)-3-[8-[(2,4-Difluorophenyl)amino]-5-oxo-5H-dibenzo[a,d]cycloheptene-3-yloxy]-1,2-propanediol), and the like, for example. Of these, SB203580, SB202190, BIRB796, LY2228820, VX-702, PH-797804, TAK-715, VX-745, and Skepinone-L are preferred. A sufficient effect can be obtained by using any of SB203580, SB202190, BIRB796, LY2228820, VX-702, PH-797804, TAK-715, VX-745, and Skepinone-L; however, SB203580, SB202190 are more preferable and SB203580 is even more preferable. These may be used alone, or two or more kinds may be used in combination.

The concentration of the p38 inhibitor in the mesenchymal stem cell culture medium of the present disclosure is preferably 1 nM - 1 µM, more preferably 10 nM - 500 nM, and further preferably 50 nM - 250 nM,

### Wnt signal activator

The Wnt signal activator in the present disclosure refers to all substances that activate the Wnt signaling. Wnt is a secretory intercellular signal transduction protein which is involved in intercellular signal transduction. This signal transduction pathway regulates cell proliferation and differentiation, mobility, functions such as body axis formation and organ formation at early embryonic development. The Wnt signaling pathway includes an intracellular signal transduction mechanism that is each individually activated by Wnt acting on the cells. As Wnt signaling pathway, the following is known: β-catenin pathway regulating the gene expression via β-catenin; PCP (planar cell polarity, the plane cell polarity) pathway regulating the planar cell polarity of the cells, Ca2⁺ pathway which promotes the intracellular mobilization of Ca2⁺, and the like. In the present specification, the Wnt signal activator, may be one which activates any of these pathways.

The Wnt signal activator includes both catenin-dependent activator such as Wnt-3a and catenin-independent activator such as Wnt-5 a . In addition to them, lithium chloride (LiCl) and complement molecule C1q and the like can be used. Of these, Wnt-3a, Wnt-5a, LiCl, and complement molecule C1q is preferable and a sufficient effect can be obtained by using any of them; however, LiCl and complement molecule C1q are more preferable, and LiCl is even more preferable. These may be used alone, or two or more kinds may be used in combination.

The concentration of the Wnt signal activator in the mesenchymal stem cell culture medium of the present disclosure is preferably 1 1µM - 10 mM, more preferably 10 µM - 10 mM, and further preferably 100 µM - 1 mM, and particularly preferably 100 µM - 500 µM,

### ROCK inhibitors

The ROCK inhibitor of the present disclosure refers to all substances inhibiting the action of Rho kinase (ROCK). Rho kinase (ROCK) is a serine/threonine protein kinase identified as a target protein of Rho which is a low molecule weight GTP binding protein. Rho kinase is involved in the physiological functions such as contraction of muscle and the like, cell proliferation, cell migration and induction of the expressions of other genes.

The ROCK inhibitor includes Y-27632 [(R)-(+)-trans-N-(4-pyridyl)-4-(1-aminoethyl)-cyclohexanecarboxamide· 2HCl· H₂O], K-115 (Ripasudil Hydrochloride Hydrate), Fasudil hydrochloride (Fasudil hydrochloride; [HA1077/1-(5-Isoquinolinesulfonyl)homopiperazine Hydrochloride]), and the like, for example. Of these, Y-27632, K-115, and Fasudil hydrochloride are preferable, and a sufficient effect can be obtained by using any of these; however, Y-27632 is more preferable.

The concentration of ROCK inhibitor in the mesenchymal stem cell culture medium of the present disclosure is preferably 1 nM - 10 µM, more preferably 10 nM - 1 µM, and further preferably 50 nM - 500 nM,

The mesenchymal stem cell culture medium of the present disclosure contains at least 2 kinds of components selected from the group consisting of a PTEN inhibitor, a p53 inhibitor, a p38 inhibitor, a Wnt signal activator, and a ROCK inhibitor; however, preferably any 3 kinds of components are contained, more preferably any 4 kinds of components are contained, and further preferably all 5 kinds of components are contained. The combination of the above-mentioned 2 kinds of components may be a combination of any of the above-mentioned 5 kinds of components and the following combination is provided in particular: a PTEN inhibitor and a p53 inhibitor; a PTEN inhibitor and a p38 inhibitor; a PTEN inhibitor and a Wnt signal activator; a PTEN inhibitor and a ROCK inhibitor; a p53 inhibitor and a p38 inhibitor; a p53 inhibitor and a Wnt signal activator; a p53 inhibitors and a ROCK inhibitor; a p38 inhibitor and a Wnt signal activator; a p38 inhibitor and a ROCK inhibitor; and a Wnt signal activator and a ROCK inhibitor. The combination of the above-mentioned 3 kinds of components may be a combination of any of the above-mentioned 5 kinds of components and the following combination is provided in particular: a PTEN inhibitor, a p53 inhibitor, and a p38 inhibitor; a PTEN inhibitor, a p53 inhibitor, and a Wnt signal activator; a PTEN inhibitor, a p53 inhibitor, and a ROCK inhibitor; a PTEN inhibitor, a p38 inhibitor, and a Wnt signal activator; a PTEN inhibitor, a p38 inhibitor, and a ROCK inhibitor; a PTEN inhibitor, a Wnt signal activator, and a ROCK inhibitor; a p53 inhibitor, a p38 inhibitor, and a Wnt signal activator; a p53 inhibitor, a p38 inhibitor, and a ROCK inhibitor; a p53 inhibitor, a Wnt signal activator, and a ROCK inhibitor; a p38 inhibitor, a Wnt signal activator, and a ROCK inhibitor. The combination of the above-mentioned 4 kinds of components may be a combination of any of the above-mentioned 5 kinds of components and the following combination is provided in particular: a PTEN inhibitor, a p53 inhibitor, a p38 inhibitor, and a Wnt signal activator; a PTEN inhibitor, a p53 inhibitor, a p38 inhibitor, and a ROCK inhibitor; a PTEN inhibitor, a p53 inhibitor, a Wnt signal activator, and a ROCK inhibitor; a PTEN inhibitor, a p38 inhibitor, a Wnt signal activator, and a ROCK inhibitor; a p53 inhibitor, a p38 inhibitor, a Wnt signal activator, and a ROCK inhibitor.

### Growth factors

As a growth factor in the stem cell culture medium of the present disclosure any growth factor known to those skilled in the art can be used. Typically, transforming growth factor (TGF), epidermal cell growth factor (EGF), and the like are provided; however, it is not limited thereto. Moreover, insulin-like growth factor (IGF), nerve growth factor (NGF), brain-derived neurotrophic factor (BDNF), vascular endothelial growth factor (VEGF), granulocyte-colony stimulating factor (G-CSF), granulocyte-macrophage colony stimulating factor (GM-CSF), platelet-derived growth factor (PDGF), erythropoietin (EPO), thrombopoietin (TPO), basic fibroblast growth factor (bFGF or FGF2), hepatocyte growth factor (HGF), and the like are provided. Further albumin, transferrin, lactoferrin, fetuin, and the like, are also exemplified. These may be used alone, or two or more kinds may be used in combination.

As the concentration of the growth factor in the mesenchymal stem cell culture medium, a concentration appropriate and adequate depending on the kind of growth factor is employed. In general, it is preferably 1 nM - 100 mM, more preferably 10 nM - 10 mM,

### Steroidal compound

As a steroidal compound in the stem cell culture medium of the present disclosure any steroidal compound known to those skilled in the art can be used. Typically, steroid hormones such as estradiol, progesterone, testosterone, cortisone, cortisol, hydrocortisone, can be used; however, it is not limited thereto. These may be used alone, or two or more kinds may be used in combination.

As the concentration of the steroidal compound in the mesenchymal stem cell culture medium a concentration appropriate and adequate depending on the kind of steroidal compound is employed. In general, it is preferably 0.1 nM - 1 mM, more preferably 1 nM - 100 µM, and more preferably 10 nM - 1 µM,

### Basal medium for culturing animal cells

Basal medium for culturing animal cells of the present disclosure refers to a medium containing a carbon source, nitrogen source, inorganic salts, and the like, essential for culturing animal cells. Here, animal cells refer to mammalian cells, in particular, human cells. Considering the possibility that the cells obtained by culturing and the culture supernatants are used for the treatment of animal (including human) diseases, the basal medium for culturing animal cells as far as possible, is preferred to be a medium which does not contain ingredients of a biological origin (e.g., serum-free medium). To the basal medium for culturing animal cells, substances that are effective at a very small amount such as micronutrient promoting substances and precursor substances can be formulated, as necessary. As such basal medium for culturing animal cells, medium for culturing animal cells known to those skilled in the art would be used. More specifically, minimal essential medium (MEM) such as Eagle's medium, Dulbecco's modified Eagle's medium (DMEM), minimum essential medium α (MEM-α), mesenchymal cell basal medium (MSCBM), Ham's F-12 and F-10 medium, DMEM/F12 medium, William's medium E, RPMI-1640 medium, MCDB medium, 199 medium, Fisher medium, Iscove's Modified Dulbecco's Medium (IMDM), McCoy's modified medium, and the like, and a mixed medium of these, and the like, are included. When used as an animal cell culture medium, in particular, DMEM/F12 medium is preferably used; however, it is not limited thereto.

To the basal medium for culturing animal cells, additives such as amino acids, inorganic salts, vitamins, carbon sources, and antibiotics can be added. The concentration for using these additives is not particularly limited, and a concentration which is used in a normal medium for culturing animal cells would be used.

Amino acids include glycine, L-alanine, L-arginine, L-asparagine, L-aspartic acid, L-cysteine, L-cystine, L-glutamic acid, L-glutamine, L-histidine, L-isoleucine, L-leucine, L-lysine, L-methionine, L-phenylalanine, L-proline, L-serine, L-threonine, L-tryptophan, L-tyrosine, L-valine, and the like, for example.

Inorganic salts include calcium chloride, copper sulfate, iron nitrate (III), iron sulfate, magnesium chloride, magnesium sulfate, potassium chloride, sodium bicarbonate, sodium chloride, disodium hydrogen phosphate, sodium dihydrogen phosphate, and the like, for example.

Vitamines include choline, vitamin A, vitamin B1, vitamin B2, vitamin B3, vitamin B4, vitamin B5, vitamin B6, vitamin B7, vitamin B12, vitamin B13, vitamin B15, vitamin B17, vitamin Bh, vitamin Bt, vitamin Bx, vitamin C (ascorbic acid), vitamin D, vitamin E, vitamin F, vitamin K, vitamin M, vitamin P, and the like, for example.

In addition, antibiotics such as penicillin, streptomycin, gentamycin, and kanamycin; carbon sources such as glucose, galactose, fructose, and sucrose; trace metals such as magnesium, iron, zinc, calcium, potassium, sodium, copper, selenium, cobalt, tin, molybdenum, nickel, and silicon; stem cell differentiation inducing agent such as β-glycerophosphate, dexamethasone, rosiglitazone, isobutyl methylxanthine, and 5-azacytidine; antioxidants such as 2-mercaptoethanol, catalase, superoxide dismutase, and N-acetylcysteine; adenosine 5'-monophosphate, corticosterone, ethanolamine, insulin, reduced glutathione, lipoic acid, melatonin, hypoxanthine, phenol red, progesterone, putrescine, pyruvic acid, thymidine, triiodothyronine, transferrin, lactoferrin, albumin, bovine serum-derived Fetuin, sodium hydrogen carbonate, buffering agents such as HEPES, Lipid mixture, an ITSE (insulin, transferrin, selenium, and ethanol amine) mixture, and the like, may be added.

The mesenchymal stem cell culture medium of the present disclosure includes the following, for example:
A DMEM/F-12 medium to which the following is added: L-glutamine, ascorbic acid, human recombinant albumin, bovine serum-derived Fetuin, sodium hydrogen carbonate, HEPES, Lipid mixture, ITSE mixture, transferrin, bFGF, progesterone, hydrocortisone, VO-OHPic, pifithrin-a (pifithrin-a), SB203580, lithium chloride, and Y-27632;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, VO-OHPic, pifithrin-a (pifithrin-a), and SB203580;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, lithium chloride and Y-27632;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, VO-OHPic, pifithrin-a (pifithrin-a), SB203580 and Y-27632;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, VO-OHPic, pifithrin-a (pifithrin-a), SB203580, and lithium chloride;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, VO-OHPic, SB203580, lithium chloride, and Y-27632;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, pifithrin-a (pifithrin-a), SB203580, lithium chloride, and Y-27632;
A DMEM/F-12 medium to which the following is added: L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone, VO-OHPic, pifithrin-a (pifithrin-a), lithium chloride, and Y-27632, and
A DMEM/F-12 medium to which the following is added: a basal medium containing L-glutamine, ascorbic acid, human recombinant albumin, bovine serum-derived Fetuin, sodium bicarbonate, HEPES, Lipid mixture, ITSE mixture, transferrin, bFGF, progesterone, hydrocortisone to which VO-OHPic and pifithrin-a (pifithrin-α); or VO-OHPic and SB203580; or VO-OHPic and lithium chloride; or VO-OHPic and Y-27632; or pifithrin-a (pifithrin-a) and SB203580; or pifithrin-a (pifithrin-a) and lithium chloride; or pifithrin-a (pifithrin-a) and Y-27632; or SB203580 and lithium chloride; or SB203580 and Y-27632; or lithium chloride and Y-27632, or the like, are added.

The method for preparing the medium is not particularly limited and it can be prepared according to the conventionally known methods. For example, the medium can be obtained by adding or mixing with each of the above-mentioned component to the basal medium for culturing animal cells at room temperature or by heating, if necessary.

The medium is preferably a liquid; however, if necessary, it can be made into a gel form or a solid medium such as agar medium. By using the medium, mesenchymal stem cells can be seeded and incubated in a culture container or culture support of which the surface of the culture surface has not undergone surface treatment.

### Methods for culturing the mesenchymal stem cells

The present invention also includes a method for culturing mesenchymal stem cells comprising culturing mesenchymal stem cells using a mesenchymal stem cell culture medium of the present disclosure. The culture method is not particular limited as long as the method for culturing uses the mesenchymal stem cell culture medium of the present disclosure, and a method alike the conventional method is used. In general, it is carried out at a temperature between 30°C to 37°C, under 2 to 7% CO₂ environment, and under 5 to 21% O₂ environment. Furthermore, the timing and method of the passage of mesenchymal stem cells is not particularly limited as long as it is suitable for each mesenchymal stem cell, and while observing the morphology of the mesenchymal stem cells, it can be carried out alike the conventional methods.

### Mesenchymal stem cells

The present disclosure comprises mesenchymal stem cells that are CD29, CD73, CD90, CD105, and CD166 positive, obtained by culturing in the mesenchymal stem cell culture medium of the present disclosure. The mesenchymal stem cells obtained by culturing in the mesenchymal stem cell culture medium maintains the undifferentiating property and strongly expresses CD29, CD73, CD90, CD105 and CD166. Furthermore, mesenchymal stem cells obtained by culturing in mesenchymal stem cell culture medium of the present disclosure have a high viability, and thus, are in a favorable condition. Furthermore, as an unexpected effect, the mesenchymal stem cells obtained by culturing in mesenchymal stem cell disclosure culture medium of the present disclosure demonstrate high resistanceto oxidative stress. Therefore, mesenchymal stem cells of the present disclosure can be effectively used in the treatment of various diseases as a medicament.

Diseases to which the mesenchymal stem cells of the present disclosure can be used as a medicament include, cartilage degradation, rheumatoid arthritis, psoriatic arthritis, spondyloarthritis, osteoarthritis, gout, psoriasis, multiple sclerosis, amyotrophic lateral sclerosis, Alzheimer's disease, Parkinson's disease, congestive heart failure, stroke, aortic valve stenosis, renal failure, lupus, pancreatitis, allergies, fibrosis, anemia, atherosclerosis, restenosis, chemotherapy/radiation-related complications, type I diabetes mellitus, type II diabetes mellitus, autoimmune hepatitis, hepatitis C, primary biliary cirrhosis, primary sclerosing cholangitis, fulminant hepatitis, celiac disease, non-specific colitis, allergic conjunctivitis, diabetic retinopathy, Sjogren's syndrome, uveitis allergic rhinitis, asthma, asbestosis, silicosis, chronic obstructive pulmonary disease, chronic granulomatous inflammation, cystic fibrosis, sarcoidosis, glomerulonephritis, vasculitis, dermatitis, HIV-related cachexia, cerebral malaria, ankylosing spondylitis, leprosy, pulmonary fibrosis, esophageal cancer, gastroesophageal reflux disease, Barrett's esophagus, gastric cancer, duodenal cancer, small intestine cancer, appendix cancer, colorectal cancer, colon cancer, rectal cancer, anal cancer, pancreatic cancer, liver cancer, gallbladder cancer, spleen cancer, kidney cancer, bladder cancer, prostate cancer, testicular cancer, uterine cancer, ovarian cancer, breast cancer, lung cancer, thyroid cancer, fibromyalgia, and the like, for example.

The administration method of the mesenchymal stem cells of the present disclosure when used as a medicament is not particularly limited; however, intravascular administration (preferably intravenous administration), intraperitoneal administration, intraintestinal administration, subcutaneous administration, and the like, are preferable, and among them, intravascular administration is more preferable.

The dosage of the mesenchymal stem cells of the present disclosure when used as a medicament may vary depending on the kind of disease, the degree of the symptom, dosage form, and weight of the subject of administration, and the like; however, the mesenchymal stem cells can be administered in a range from 1 x 10⁵ to 1 x10⁹ per day. Furthermore, administration of the mesenchymal stem cells when used as a medicament can be carried out in a single or divided dose(s) throughout the day. Furthermore, the above-mentioned administration can be a single dose administration or a continuous administration. When the administration is carried out continuously, it can be continuously administered at a frequency of once or more in 3 days for two or more occasions.

Mammals that can be the subject of administration when mesenchymal stem disclosure cells of the present disclosure are used as a medicament are not particularly limited, but human, monkey, mouse, rat, hamster, guinea pig, cow, pig, horse, rabbit, sheep, goat, cat, dog, and the like, are preferable, and among them, human is more preferable. Furthermore, when mesenchymal stem cells are used as a medicament, it is preferable that the kind of mammal (from which the mesenchymal stem cells derived) is consistent with the kind of the mammal as the subject of administration, from the viewpoint of obtaining a further stable and excellent prophylactic and/or therapeutic effect against the disease.

The present invention includes methods for preparing medicaments containing the mesenchymal stem cells of the present disclosure mentioned above. The method for preparing the medicament comprising the mesenchymal stem cells comprises the processes for preparing mesenchymal stem cells according to the method for culturing the above-mentioned mesenchymal stem cells and the processes for preserving the obtained mesenchymal stem cells in a pharmaceutically acceptable preservation solution. Furthermore, the method for preparing the medicament comprising the mesenchymal stem cells may further comprise suspending the mesenchymal stem cells of the present invention in a buffer solution and the like for administration, as necessary.

### EXAMPLES

Hereinafter, the disclosure will be further described in detail with reference to the Examples,

### Preparation of the mesenchymal stem cell culture medium

14-2-1 medium with a basic formulation as mentioned below in Table 1 (Comparative Example 1) was prepared. More specifically, the components provided in the below-mentioned Table 1 were added to a DMEM/F12 medium at a concentration given below.

**Table 1**

| Components | Concentration |
|---|---|
| DMEM/F-12 | - |
| L-Glutamine | 4 mM |
| Ascorbic acid | 50 ug/ml |
| Human recombinant Albumin | 4 mg/ml |
| Bovine Fetuin | 1 mg/ml |
| NaHCO3 | 20.5 mM |
| HEPES | 4.9 mM |
| Lipids (Chemically Defined Lipid Concentrate) | 0.1 % (v/v) |
| ITSE | Insulin: 10 ug/ml, Transferrin: 5.5 ug/ml, Sodium selenite:6.7 ng/ml, Ethanolamine:2 ug/ml |
| bFGF | 2 ng/ml |
| Progesterone | 0.018 uM |
| Hydrocortisone (50 uM) | 100 nM |

To the 14-2-1 medium prepared as mentioned above, each component shown below in Table 2 was added at a concentration given below for the preparation of each medium (Comparative Example 1 and Examples 1 to 8). This medium was provided for culturing the umbilical cord mesenchymal stem cells and adipose derived mesenchymal stem cells.

### Table 2

**Table 2**

| Additive components | | Concentration | Comparative Example 1 | Example 1 | Example 2 | Example 3 | Example 4 | Example 5 | Example 6 | Example 7 | Example 8 |
|---|---|---|---|---|---|---|---|---|---|---|---|
| LiCl | Wnt activator | 250 uM | - | + | - | + | - | + | + | + | + |
| Y-27632 | ROCK inhibitor | 100 nM | - | + | - | + | + | - | + | + | + |
| Pifithrin-a | p53 inhibitor | 10 uM | - | + | + | - | + | + | - | + | + |
| VO-OH Pic | PTEN inhibitor | 500 nM | - | + | + | - | + | + | + | - | + |
| SB203580 | p38 inhibitor | 100 nM | - | + | + | - | + | + | + | + | - |

### Culture and assessment of the umbilical cord mesenchymal stem cells and adipose derived mesenchymal stem cells

### (Test 1)

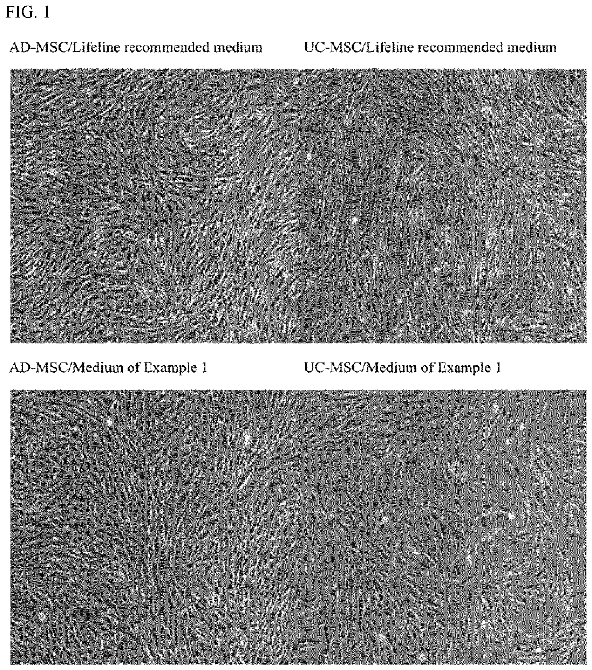

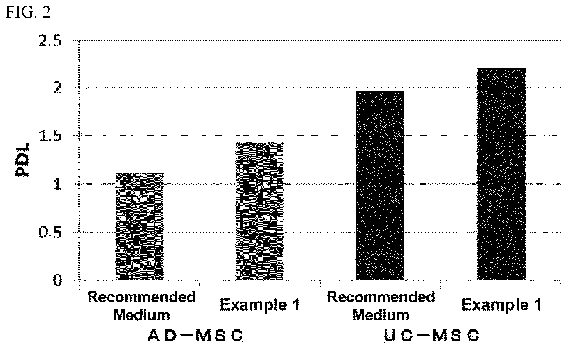

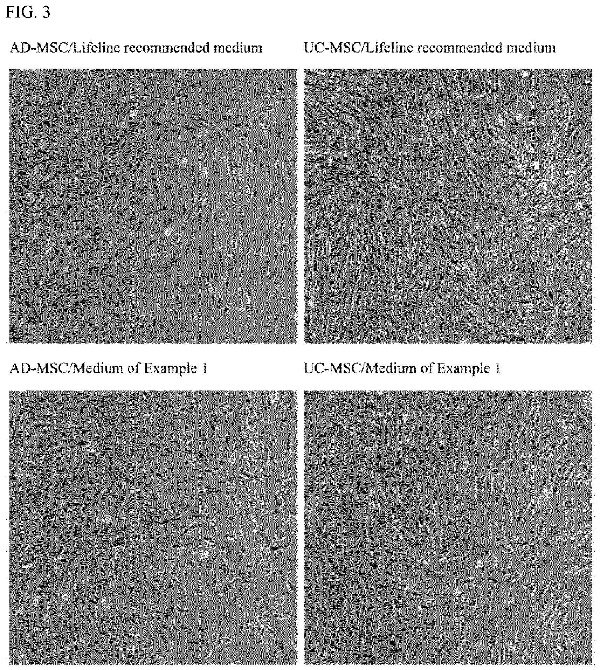

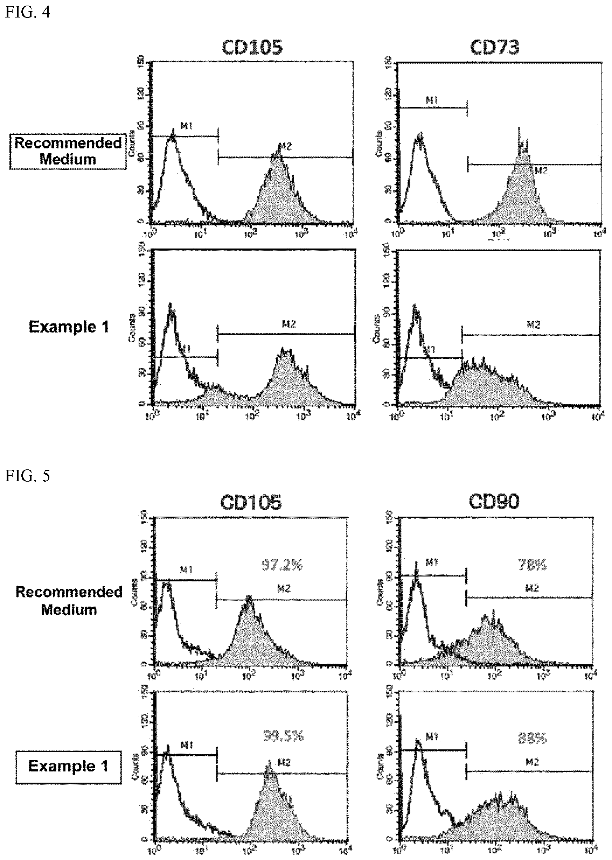

Umbilical cord derived mesenchymal stem cells (UC-MSC; Umbilical Cord derived Mesenchymal Stem Cells Wharton's Jelly(HMSC-WJ), FC-0020, LIFELINE CELL TECHNOLOGY Co., Ltd.) and adipose derived mesenchymal stem cells (AD-MSC; Adipose derived Mesenchymal Stem Cells, FC-0034, LIFELINE CELL TECHNOLOGY Co., Ltd.) were conditioned with a recommended medium of LIFELINE CELL TECHNOLOGY Co., Ltd. at 37°C under 5% of CO₂, and then seeded to CellBind Flask at a density of 5,000 cells/cm². The next day, this medium was exchanged with the recommended medium of LIFELINE CELL TECHNOLOGY Co., Ltd. or the medium of Example 1 (1 X 10⁵ cells/well; 6 well plate). 3 days later, each of them were seeded again in the same way, and then, after another 3 days (in total, after 6 days), the number of cells were counted. The morphological change and the cell surface marker expression were analyzed for the cells cultured for another 2 days (in total 8 days). The growth rate (the number of the division of the seeded cells until the next passage) until 8 days is shown in Fig. 2. Moreover, the cell photographs after 3 days and 8 days are shown in Fig. 1 and Fig. 3, respectively. Furthermore, the cells after 8 days have been analyzed for the expression of cell surface markers (CD105 and CD73 for adipose derived mesenchymal stem cells and CD105 and CD90 for umbilical cord derived mesenchymal stem cells) using FACS, and the results are shown in Fig. 4 and Fig. 5.

AD-MSC exhibited a better proliferation capacity when cultured in the medium of Example 1 as compared to the recommended medium (Fig. 2). Moreover, as for the morphology of the cells, not much difference was observed until day 3 of culturing (Fig. 1). Nonetheless, by day 8 (Fig. 3), the cells cultured in the medium of Example 1 were somewhat smaller and round and in a favorable condition, whereas the cells cultured in the recommended medium had an elongated morphology and were suggested to be in a rather unfavorable condition. With respect to the expression of the surface markers, whereas AD-MSC cultured in a recommended medium demonstrated a high and single expression peak of CD105 and CD73, the AD-MSC cultured in the medium of Example 1 showed a low expression of CD105 in some of the cells, and the expression peak was split into two. Moreover, as for the CD73 expression, the overall expression signal was weaker. For this reason, the recommended medium was suggested to be more suitable for the maintenance of the undifferentiating property of AD-MSC.

On the other hand, as for the UC-MSC, the medium of Example 1 demonstrated a better proliferation on day 6 of culturing. As for the morphology of the cells, as shown in Fig. 1 and Fig. 3, the medium of Example 1 demonstrated a better condition as the cells were smaller and round. Moreover, as for the expression of the surface markers, the cells which were cultured in the medium of Example 1 demonstrated a higher expression peak of CD105 and CD90 and the medium of Example 1 was determined to better maintain the undifferentiating property.

### (Test 2)

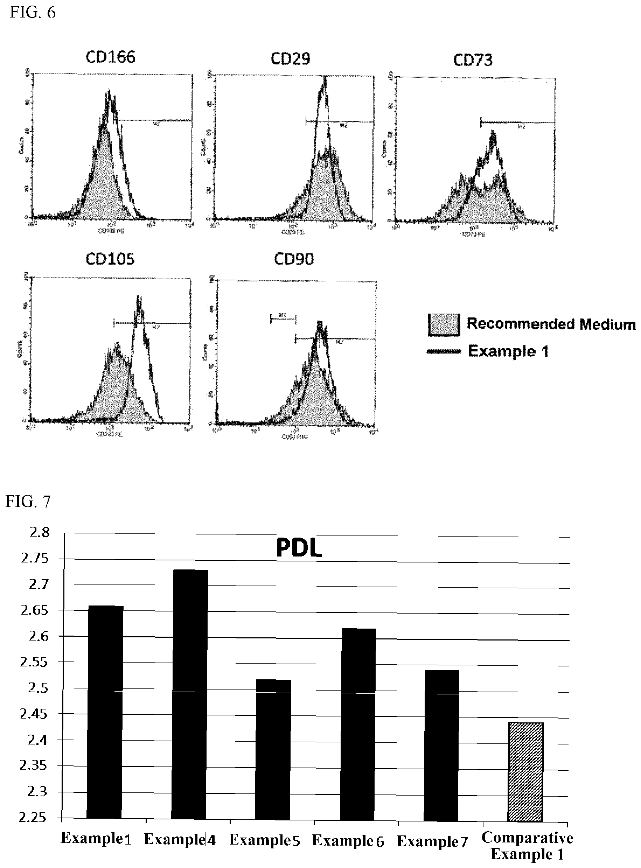

Umbilical cord derived mesenchymal stem cells (UC-MSC; Umbilical Cord derived Mesenchymal Stem Cells Wharton's Jelly (HMSC-WJ), FC-0020, LIFELINE CELL TECHNOLOGY Co., Ltd.) was conditioned with a recommended medium of LIFELINE CELL TECHNOLOGY Co., Ltd. at 37°C under 5% of CO₂, and then this medium was exchanged with the recommended medium of LIFELINE CELL TECHNOLOGY Co., Ltd. or the medium of Example 1 (1 X 10⁵ cells/well; 6 well plate). The passage was carried out every 2 to 3 days and cells at day 11 from the exchange of medium were subjected for the analysis of the expression of the cell surface markers (CD29. CD73, CD90, 105, and CD166) using FACS. The results are shown in Fig. 6.

As shown in Fig. 6, with respect to the cell surface markers of US-MSC, the cell cultured in the medium of Example 1 showed a higher expression of CD73, CD90, 105, and CD166 than the cells cultured in the recommended medium, and the peak of CD29 was more uniformed. Accordingly, it was determined that cells cultured in the medium of Example 1 would better maintain the undifferentiating property of UC-MSC.

### (Test 3)

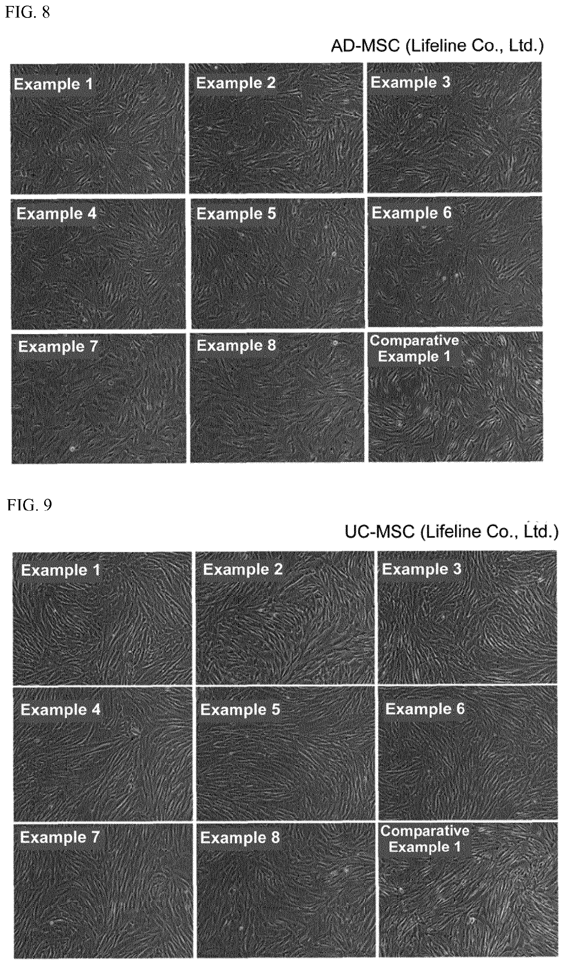

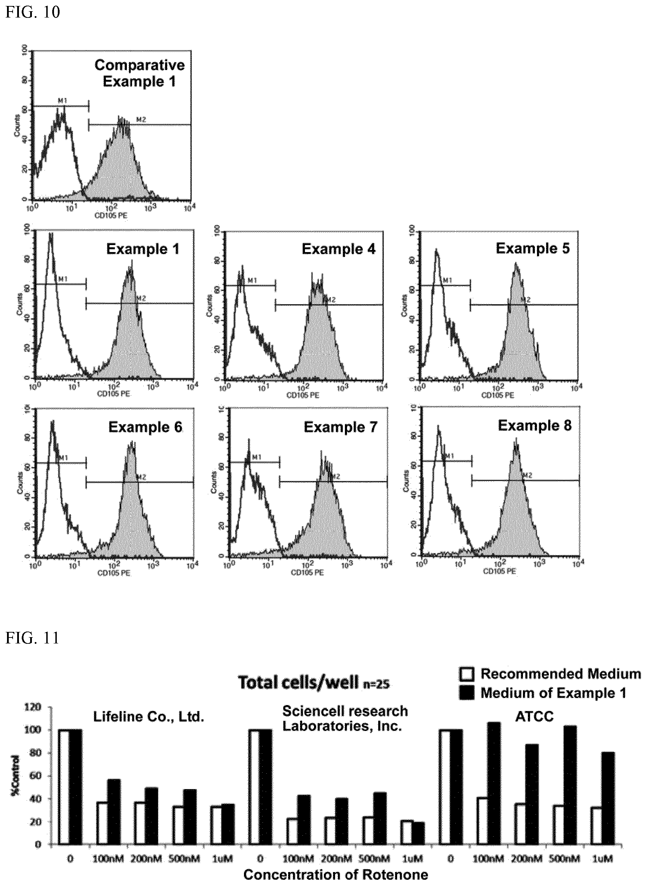

Umbilical cord derived mesenchymal stem cells (UC-MSC; Umbilical Cord derived Mesenchymal Stem Cells Wharton's Jelly(HMSC-WJ), FC-0020, LIFELINE CELL TECHNOLOGY Co., Ltd.) and adipose derived mesenchymal stem cells (AD-MSC; Adipose derived Mesenchymal Stem Cells, FC-0034, LIFELINE CELL TECHNOLOGY Co., Ltd.) were conditioned with a recommended medium of LIFELINE CELL TECHNOLOGY Co., Ltd. at 37°C under 5% of CO₂, and then, each of them were cultured in each medium described in Table 2 for 5 days (70,000 cells/well at the time of seeding). Subsequently, the number of cells was counted and the results are shown as PDL in Fig. 7. The cell photographs are shown in Fig. 8 and Fig. 9. Furthermore, the passages were carried out during culturing, and the expression of the cell surface marker (CD105) of the cells cultured in each medium in a total of 3 weeks was analyzed using FACS. The results are shown in Fig. 10

The medium of Example 1 would favorably maintain the morphology of the cells for both AD-MSC and UC-MSC as compared to the medium of comparative Example 1. As for the surface marker of UC-MSC, the medium of Example 1 showed a higher expression of CD105, the peak was sharper, and thus, it is understood that an undifferentiating and uniform population would be maintained.

The medium of Examples 2 and 3 significantly promoted the proliferation of UC-MSC as compared to that of AD-MSC. The morphology of the cells was favorable in both AD-MSC and UC-MSC.

The medium of Example 4 further promoted the proliferation of UC-MSC as compared to that of AD-MSC. The morphology of the cells was favorable in both AD-MSC and UC-MSC. However, the width of the peak of the surface marker (CD105) of UC-MSC was rather wide and the uniformity of the cell population was somewhat reduced.

The medium of Examples 5 and 6 further promoted the proliferation of UC-MSC as compared to that of AD-MSC. The morphology of the cells was somewhat elongated in AD-MSC but was favorable in UC-MSC. As for the surface marker of UC-MSC (CD105), a sharp peak was shown, and thus, it is understood that an undifferentiating and uniform population would be maintained.

The medium of Example 7 significantly promoted the proliferation of UC-MSC as compared to that of AD-MSC. The morphology of the cells was favorable in both AD-MSC and UC-MSC. However, the width of the peak of the surface marker (CD105) of UC-MSC tended to be rather wide.

The medium of Example 8 significantly promoted the proliferation of UC-MSC as compared to that of AD-MSC. The morphology of the cells was favorable in both AD-MSC and UC-MSC. As for the surface marker of UC-MSC (CD105), a sharp peak was shown, and thus, it is understood that an undifferentiating and uniform population would be maintained.

As stated above, a medium containing any 4 kinds of a PTEN inhibitor, a p53 inhibitor, a p38 inhibitor, and a Wnt signal activator, and a ROCK inhibitor (Examples 4 to 8) would allow the cells to be cultured while maintaining the cell morphology in a favorable condition in both AD-MSC and UC-MSC. In addition, this medium tended to further promote the proliferation of UC-MSC as compared to that of the AD-MSC. Furthermore, it would maintain the favorable condition of UC-MSC almost to the same degree as the medium of Example 1 containing all of the above-mentioned 5 components, as well as the cells would be cultured while maintaining the undifferentiating property. Moreover, the medium of Example 2 containing a PTEN inhibitor, a p53 inhibitor, and a p38 inhibitor, the medium of Example 3 containing a Wnt signal activator and a ROCK inhibitor also had an effect of promoting the proliferation of both AD-MSC and UC-MSC while maintaining a favorable morphology, in particular, the proliferation of UC-MSC.

### (Test 4) Induction of oxidative stress resistance

(UC-MSC; Umbilical Cord derived Mesenchymal Stem Cells Wharton's Jelly (HMSC-WJ), FC-0020, LIFELINE CELL TECHNOLOGY Co., Ltd.; UC-MSC; Umbilical Cord derived Mesenchymal Stem Cells ScienCell Research Laboratories, Inc; and Umbilical Cord derived Mesenchymal Stem Cells ATCC) were conditioned with a recommended medium of each entity at 37°C under 5% of CO₂, and they were exchanged with the recommended medium of each entity or the medium of Example 1 (0.3 - 1 x 10⁵ cells/well; 6 well plate). The passage was carried out every 2 to 3 days and those cells were treated with Rotenone of the following concentrations (0 nM, 100 nM, 200 nM, 500 nM, 1 µM). After 48 hours, these cells were stained with Hoechest33258 and the number of nucleus was counted using ImageXpress (Molecular Devices, LLC.). The results are shown in Fig. 11.

As shown in Fig. 11, the cell number of UC-MSC decreases in a concentration dependent manner of the Rotenone treatment as a result of being damaged. However, it was suggested that culturing in the medium of Example 1 would suppress the decrease of the cell numbers, and granted resistancy against the damage caused by Rotenone and the cells are at a state less affected by oxidative stress.

### (Test 5) Long-term Culture 1

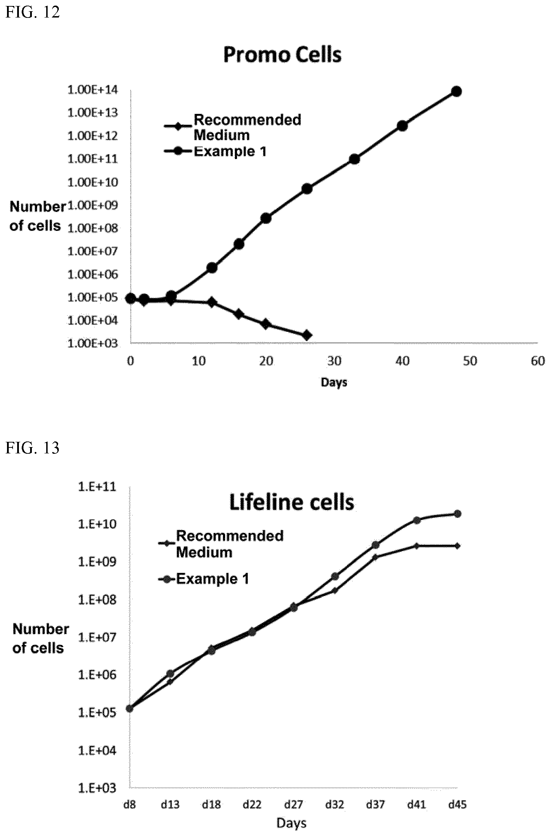

Umbilical cord derived mesenchymal stem cells (UC-MSC; Umbilical Cord derived Mesenchymal Stem Cells, c-12971, PromoCell, Ltd.) were conditioned with a recommended medium of PromoCell, Ltd. at 37°C under 5% of CO₂, and passage culture was carried out over a long period of time in Promo Media (c-39810 or c-28019, PromoCell, Ltd.) and in the medium of Example 1 of Table 2. The results of the total number of cells counted are shown in Fig. 12.

Culturing in the medium of Example 1 demonstrated both favorable morphology and proliferation ability of the cells even when passage culturing was continued until P12 (50 days). On the other hand, the cells in Promo Media which is a commercial medium would not be maintained after P9.

### (Test 6) Long-term Culture 2

Umbilical cord derived mesenchymal stem cells (UC-MSC; Umbilical Cord derived Mesenchymal Stem Cells Wharton's Jelly (HMSC-WJ), FC-0020, LIFELINE CELL TECHNOLOGY Co., Ltd.) were conditioned with the recommended medium of LIFELINE CELL TECHNOLOGY Co., Ltd. at 37°C under 5% of CO₂, and passage culture was carried out over a long period of time in the medium of Example 1 of Table 2 or a recommended medium of LIFELINE CELL TECHNOLOGY Co., Ltd. The results of the total number of cells counted are shown in Fig. 13.

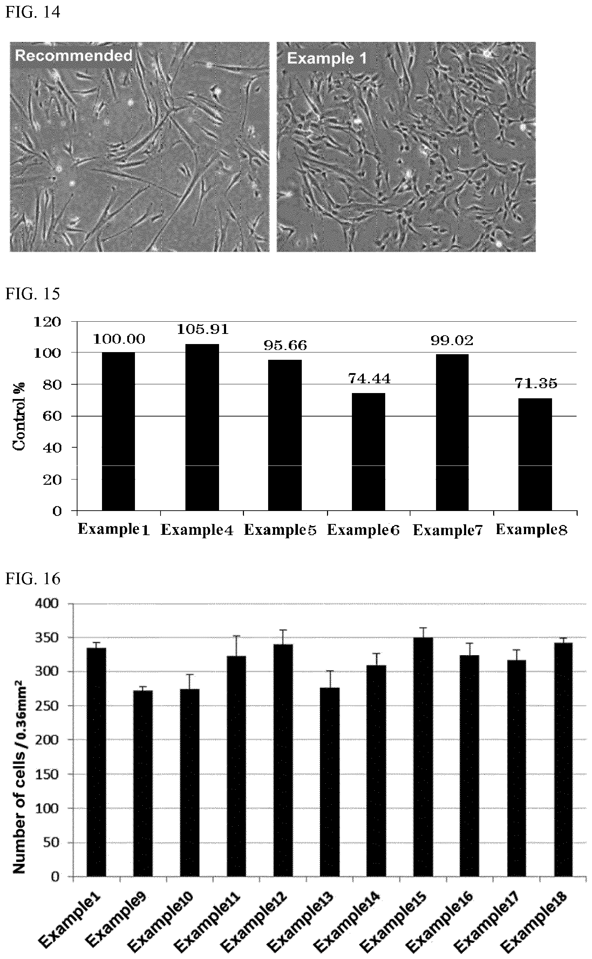

Culturing in the medium of Example 1 demonstrated both favorable morphology and proliferation ability of the cells even when passage culturing was continued for 45 days and they were comparable to those cultured in the recommended medium of LIFELINE CELL TECHNOLOGY Co., Ltd.. Note that as for the morphology of the cells, whereas the cells cultured over a long period of time (4 days after 16 passages) in the recommended medium of LIFELINE CELL TECHNOLOGY Co., Ltd. had an elongated shape, the cells cultured over a long period of time in the medium of Example 1 (on 4 days after 9 passages in the medium of Example 1 which followed 7 passages in the recommended medium of LIFELINE CELL TECHNOLOGY Co., Ltd.) kept on maintaining a favorable morphology of a spindle shape (Fig. 14). As for the total number of cells, a higher number of cells were observed when cultured in the medium of Example 1 and the medium of Example 1 is discovered to have a superior proliferation promoting effect for mesenchymal stem cells (Fig. 13).

### (Test 7)

Umbilical cord derived mesenchymal stem cells (UC-MSC; Umbilical Cord derived Mesenchymal Stem Cells Wharton's Jelly (HMSC-WJ), FC-0020, LIFELINE CELL TECHNOLOGY Co., Ltd.) were conditioned with a recommended medium of LIFELINE CELL TECHNOLOGY Co., Ltd. at 37°C under 5% of CO₂, and then the cells were cultured in the medium of Examples 1 and 4 to 8 of Table 2 for 8 days, and then the cell proliferation effect was compared. The comparison of the cell proliferation effect was carried out by staining the cells with Hoechest33342 and the images of the cells were acquired by ImageXpress (Molecular Devices, LLC.) for counting the number of cells. The results are shown in Fig. 15.

As shown in Fig. 15, as compared to the result of the medium of Example 1 (100%) containing all 5 components such as a PTEN inhibitor, a p53 inhibitor, a p38 inhibitor, a Wnt signal activator, and a ROCK inhibitor (100%), using the medium of Examples 6 and 8 which do not contain a p53 inhibitor or a p38 inhibitor would allow obtaining an equivalent degree of UC-MSC proliferation effect despite of a minor decrease even when one component of the above-mentioned 5 components is missing.

### (Test 8)

Umbilical cord derived mesenchymal stem cells (UC-MSC; Umbilical Cord derived Mesenchymal Stem Cells Wharton's Jelly (HMSC-WJ), FC-0020, LIFELINE CELL TECHNOLOGY Co., Ltd.) were conditioned with a recommended medium of LIFELINE CELL TECHNOLOGY Co., Ltd. at 37°C under 5% of CO₂, and then the cells were cultured in the medium of Examples 9 to 18 of the below-mentioned Table 3 and the medium of Example 1 of the above-mentioned Table 2 for 5 days, and then the cell proliferation effect was compared. The comparison of the cell proliferation effect was carried out by staining the cells with Hoechest33342 and the images of the cells were acquired by ImageXpress (Molecular Devices, LLC.) for counting the number of cells (Number of cells/0.36 mm²). The results are shown in Fig. 16.

### Table 3

**Table 3**

| Additive components | | Concentration | Example 1 | Example 9 | Example 10 | Example 11 | Example 12 | Example 13 | Example 14 | Example 15 | Example 16 | Example 17 | Example 18 |
|---|---|---|---|---|---|---|---|---|---|---|---|---|---|
| LiCl | Wnt activator | 250 uM | + | + | + | + | + | - | - | - | - | - | - |
| Y-27632 | ROCK inhibitor | 100 nM | + | + | - | - | - | + | + | + | - | - | - |
| Pifithrin-a | p53 inhibitor | 10 uM | + | - | + | - | - | + | - | - | + | + | - |
| VO-OH Pic | PTEN inhibitor | 500 nM | + | - | - | + | - | - | + | - | + | - | + |
| SB203580 | p38 inhibitor | 100 nM | + | - | - | - | + | - | - | + | - | + | + |

As shown in Fig. 15, when the medium of Example 1 containing all of the 5 components of a PTEN inhibitor, a p53 inhibitor, a p38 inhibitor, a Wnt signal activator, and a ROCK inhibitor and a medium containing any two components of them (Examples 9 to 18) were compared, it is understood that the proliferation effect obtained for UC-MSC for 5 days would be more or less the same degree in any medium being used. Therefore, when a medium containing any 2 kinds of components of the 5 components of a PTEN inhibitor, a p53 inhibitor, a p38 inhibitor, a Wnt signal activator and a ROCK inhibitor is used, in a system culturing the UC-MSC for 5 days, a sufficient and superior proliferation promoting effect would be obtained.

## Claims

1. A mesenchymal stem cell culture medium comprising
at least 3 kinds of components selected from the group consisting of a PTEN inhibitor, a p53 inhibitor, a p38 inhibitor, an Wnt signal activator and a ROCK inhibitor, with the proviso that the at least 3 kinds of components does not consist only of a PTEN inhibitor, a p53 inhibitor and a Wnt signal activator; and
basal medium for culturing animal cells; wherein
the PTEN inhibitor is selected from the group consisting of VO-OHPic, HOPic and pV,
the p53 inhibitor is selected from the group consisting of sodium orthovanadate, pifithrin-α and MDM2 protein,
the p38 inhibitor is selected from the group consisting of SB203580, SB202190, BIRB796, LY2228820, VX-702, PH-797804, TAK-715, VX-745 and Skepinone-L,
the Wnt signal activator is a LiCl or a complement molecule C1q, and
the ROCK inhibitor is selected from the group consisting of Y-27632, K-115 and Fasudil hydrochloride.

2. The mesenchymal stem cell culture medium according to claim 1 comprising at least 4 kinds of components selected from the group consisting of a PTEN inhibitor, a p53 inhibitor, a p38 inhibitor, an Wnt signal activator and a ROCK inhibitor as well as basal medium for culturing animal cells.

3. The mesenchymal stem cell culture medium according to claim 1 or 2 comprising the PTEN inhibitor, the p53 inhibitor, the p38 inhibitor, the Wnt signal activator, and the ROCK inhibitor.

4. The mesenchymal stem cell culture medium according to any one of claims 1 to 3 further comprising at least one kind of component selected from the group consisting of growth factors and steroidal compounds.

## Patentansprüche

1. Kulturmedium für mesenchymale Stammzellen, umfassend
zumindest 3 Arten von Komponenten, ausgewählt aus der aus einem PTEN-Inhibitor, einem p53-Inhibitor, einem p38-Inhibitor, einem Wnt-Signalaktivator und einem ROCK-Inhibitor bestehenden Gruppe, mit der Maßgabe, dass die zumindest 3 Arten von Komponenten nicht nur aus einem PTEN-Inhibitor, einem p53-Inhibitor und einem Wnt-Signalaktivator bestehen; und
ein Basismedium zum Kultivieren von Tierzellen;
wobei der PTEN-Inhibitor aus der aus VO-OHPic, HOPic und pV bestehenden Gruppe ausgewählt ist,
wobei der p53-Inhibitor aus der aus Natriumorthovanadat, Pifithrin-α und MDM2-Protein bestehenden Gruppe ausgewählt ist,
wobei der p38-Inhibitor aus der aus SB203580, SB202190, BIRB796, LY2228820, VX-702, PH-797804, TAK-715, VX-745 und Skepinon-L bestehenden Gruppe ausgewählt ist,
wobei der Wnt-Signalaktivator ein LiCI-Molekül oder ein C1q-Komplementmolekül ist und
wobei der ROCK-Inhibitor aus der aus Y-27632, K-115 und Fasudilhydrochlorid bestehenden Gruppe ausgewählt ist.

2. Kulturmedium für mesenchymale Stammzellen nach Anspruch 1, umfassend zumindest 4 Arten von Komponenten, die aus der aus einem PTEN-Inhibitor, einem p53-Inhibitor, einem p38-Inhibitor, einem Wnt-Signalaktivator und einem ROCK-Inhibitor bestehenden Gruppe ausgewählt sind, sowie ein Basismedium zum Kultivieren von Tierzellen.

3. Kulturmedium für mesenchymale Stammzellen nach Anspruch 1 oder 2, umfassend den PTEN-Inhibitor, den p53-Inhibitor, den p38-Inhibitor, den Wnt-Signalaktivator und den ROCK-Inhibitor.

4. Kulturmedium für mesenchymale Stammzellen nach einem der Ansprüche 1 bis 3, weiters umfassend zumindest eine Art von Komponente, die aus der aus Wachstumsfaktoren und Steroidverbindungen bestehenden Gruppe ausgewählt ist.

## Revendications

1. Milieu de culture de cellules souches mésenchymateuses, comprenant
au moins 3 types de composants choisis dans le groupe comprenant un inhibiteur de PTEN, un inhibiteur de p53, un inhibiteur de p38, un activateur de signal Wnt et un inhibiteur de ROCK, à condition que les au moins 3 types de composants ne consistent pas uniquement en un inhibiteur de PTEN, un inhibiteur de p53 et un activateur de signal Wnt ; et
un milieu de base pour cultiver des cellules animales ; dans lequel
l'inhibiteur de PTEN est choisi dans le groupe comprenant VO-OHPic, HOPic et pV,
l'inhibiteur de p53 est choisi dans le groupe comprenant l'orthovanadate de sodium, la pifithrine-α et la protéine MDM2,
l'inhibiteur de p38 est choisi dans le groupe comprenant SB203580, SB202190, BIRB796, LY2228820, VX-702, PH-797804, TAK-715, VX-745 et Skepinone-L,
l'activateur de signal Wnt est un LiCl ou une molécule de complément C1q, et l'inhibiteur de ROCK est choisi dans le groupe comprenant Y-27632, K-115 et le chlorhydrate de Fasudil.

2. Milieu de culture de cellules souches mésenchymateuses selon la revendication 1, comprenant au moins 4 types de composants choisis dans le groupe comprenant un inhibiteur de PTEN, un inhibiteur de p53, un inhibiteur de p38, un activateur de signal Wnt et un inhibiteur de ROCK, ainsi qu'un milieu de base pour cultiver des cellules animales.

3. Milieu de culture de cellules souches mésenchymateuses selon la revendication 1, comprenant l'inhibiteur de PTEN, l'inhibiteur de p53, l'inhibiteur de p38, l'activateur de signal Wnt et l'inhibiteur de ROCK.

4. Milieu de culture de cellules souches mésenchymateuses selon l'une quelconque des revendications 1 à 3, comprenant en outre au moins un type de composant choisi dans le groupe comprenant des facteurs de croissance et des composés stéroïdiens.
